# EUROPEAN PATENT APPLICATION

(11) **EP 3 067 432 A1**
(43) Date of publication of application: **14.09.2016**
(21) Application number: 15158660.9
(22) Date of filing: 11.03.2015
(51) Int. Cl.: C12Q 1/68

(54) **DNA-methylation based method for classifying tumor species of the brain**

(71) Applicant: Deutsches Krebsforschungszentrum Stiftung des Öffentlichen Rechts, 69120 Heidelberg (DE); Ruprecht-Karls-Universität Heidelberg, 69117 Heidelberg (DE)
(72) Inventor: Pfister, Stefan, 69121 Heidelberg (DE); von Deimling, Andreas, 69198 Schriesheim (DE); Jones, David, 69115 Heidelberg (DE); Capper, David, 69115 Heidelberg (DE); Hovestadt, Volker, 69115 Heidelberg (DE); Sill, Martin, 69120 Heidelberg (DE); Bewerunge-Hudler, Melanie, 69221 Dossenheim (DE); Schick, Matthias, 69256 Mauer (DE)
(74) Representative: Krauss, Jan

(57) **Abstract**

The present invention pertains to methods for classifying tumorous diseases based on their specific genomic DNA methylation profile. The invention provides a method that allows for a classification of a tumor sample obtained from a patient by analysing a multitude, preferably genome wide, collection of CpG positions by comparison to a classification rule derived from a set of methylation data acquired from pre-classified tumor species. The invention is in particular useful for classifying brain tumor samples since brain tumors are characterized by a large variety of distinct tumor species which have different prognostic values and require in the clinic a for each species developed treatment regime.

## Description

### FIELD OF THE INVENTION

The present invention pertains to methods for classifying tumorous diseases based on their specific genomic DNA methylation profile. The invention provides a method that allows for a classification of a tumor sample obtained from a patient by analysing a multitude, preferably genome wide, collection of CpG positions by comparison to a classification rule derived from a set of methylation data acquired from pre-classified tumor species. The invention is in particular useful for classifying brain tumor samples since brain tumors are characterized by a large variety of distinct tumor species which have different prognostic values and require in the clinic a for each species developed treatment regime.

### DESCRIPTION

There are more than 100 brain tumor entities listed in the World Health Organisation classification. Many of these show complex patterns of potentially overlapping histological features. Moreover, even histologically identical tumors can belong to different molecular groups with very different treatment requirements and prognosis. Therefore more advanced diagnostic tools are needed.

Changes in DNA methylation patterns play a critical role in development, differentiation and diseases such as multiple sclerosis, diabetes, schizophrenia, aging, and multiple forms of cancer including tumors of the central nervous system. Brain tumor entities originate from different precursor-cell populations which are transformed by genetic and epigenetic alterations. It is now recognized that many brain tumor entities that are of distinct biological groups are not always distinguishable in histology. Most of the brain tumor entities display varied histological spectra with no clear boundaries. Epigenetic modifications such as methylation preserve the information of the cell of origin, it's original identity. Therefore methylation data has a great potential to identify molecular subgroups of brain tumors.

Still, treatment planning and in particular treatment success in many cancer diseases, and in particular in brain cancers, is highly dependent on an early and accurate diagnosis and classification of the tumor. Hence, the present invention seeks to provide a strategy for classification of tumor samples with higher specificity and sensitivity.

The above problem is solved in a first aspect a method for the classification, stratification and/or diagnosis of a tumor species, the method comprising the steps of
(a) Providing a tumor-sample to be classified obtained from a tumor of a patient and, optionally, isolating genomic DNA therefrom,
(b) Determining DNA methylation status of a multitude of independent genomic CpG positions in the genome of said tumor-sample, and
(c) Classifying the tumor species of the tumor-sample based on the methylation levels as determined in (b) using a classification-rule,
wherein the classification-rule is obtained by random forest analysis of a training-data-set, the training-data-set comprising pre-determined methylation data derived from multitude of pre-classified tumor species, wherein said pre-determined methylation data comprises the methylation status of said CpG positions in the genome of each of said pre-classified tumor species.

To this end the inventor's tested their approach using an Illumina methylation bead chip with which a multitude of classically classified brain tumor specimen were tested. Illumina's HumanMethylation450 (450k) BeadChip allows to assays DNA methylation at 482,421 CpG dinucleotides. The platform measures DNA methylation by genotyping sodium bisulfite treated DNA. To run the assay only a little amount of DNA is needed and it is possible to use both frozen and paraffin (FFPE) material. So far approximately 8000 thousand brain tumor samples have been profiled by the inventors, and allowed the verification of the surprisingly superior approach of the herein disclosed invention.

The term "classification" refers to a procedure and/or algorithm in which individual items are placed into groups or classes based on quantitative information on one or more characteristics inherent in the items (referred to as traits, variables, characters, features, etc.) and based on a statistical model and/or a training set of previously labeled items. A "classification tree" is a decision tree that places categorical variables into classes.

In the context of the present invention, the term "stratification" refers to the classification or grouping of patients according to one or more predetermined criteria. In certain embodiments, stratification is performed in a diagnostic setting in order to group a patient according to the prognosis of disease progression, either with or without treatment. In particular embodiments, stratification is used in order to distribute patients enrolled for a clinical study according to their individual characteristics. In particular embodiments, stratification is used in order to identify the best suitable treatment option for a patient.

The term "diagnosis" is used herein to refer to the identification or classification of a molecular or pathological state, disease or condition. For example, "diagnosis" may refer to identification of a particular type of cancer, e.g., a lung cancer. "Diagnosis" may also refer to the classification of a particular type of cancer, e.g., by histology (e.g., a non small cell lung carcinoma), by molecular features (e.g., a lung cancer characterized by nucleotide and/or amino acid variation(s) in a particular gene or protein), or both.

The term "tumor species" or "tumor class" shall refer to a specific kind of a tumor or sub category of a tumor that can be classified based on its tissue origin, genetic make up, histology etc. In particular in the field of brain tumors, various distinct brain tumor species or classes exist that can be differentiated via for example histopathology (1. Acta Neuropathol. 2007 Aug;114(2):97-109. Epub 2007 Jul 6."The 2007 WHO classification of tumours of the central nervous system." Louis DN(1), Ohgaki H, Wiestler OD, Cavenee WK, Burger PC, Jouvet A, Scheithauer BW, Kleihues P.).

The term "tumor sample" as used herein, refers to a sample obtained from a patient. The tumor sample can be obtained from the patient by routine measures known to the person skilled in the art, i.e., biopsy (taken by aspiration or punctuation, excision or by any other surgical method leading to biopsy or resected cellular material). For those areas not easily reached via an open biopsy, a surgeon can, through a small hole made in the skull, use stereotaxic instrumentation to obtain a "closed" biopsy. Stereotaxic instrumentation allows the surgeon to precisely position a biopsy probe in three-dimensional space to allow access almost anywhere in the brain. Therefore, it is possible to obtain tissue for the diagnostic method of the present invention.

The term "tumor" is not limited to any stage, grade, histomorphological feature, invasiveness, aggressiveness or malignancy of an affected tissue or cell aggregation. In particular stage 0 cancer, stage I cancer, stage II cancer, stage III cancer, stage IV cancer, grade I cancer, grade II cancer, grade III cancer, malignant cancer, primary carcinomas, and all other types of cancers, malignancies etc. are included.

As used herein the term "CpG site" or "CpG position" refers to a region of DNA where a cytosine nucleotide occurs next to a guanine nucleotide in the linear sequence of bases along its length, the cytosine (C) being separated by only one phosphate (p) from the guanine (G). About 70% of human gene promoters have a high CpG content. Regions of the genome that have a higher concentration of CpG sites are known as "CpG islands". Cytosines in CpG dinucleotides can be methylated to form 5-methylcytosine. Methylation of (i.e., introduction of a methyl group in) the cytosines of CpG site within the promoters of genes can lead to gene silencing, a feature found in a number of human cancers. In contrast, the hypomethylation of CpG sites has generally been associated with the over-expression of oncogenes within cancer cells. The term "independent genomic CpG positions" shall in the context of the present invention mean that each CpG position of a group of genomic CpG positions can be probed separately for its methylation status.

The term "methylation status", as used herein describes the state of methylation of a CpG position, thus refers to the presence or absence of 5-methylcytosine at one CpG site within genomic DNA. When none of the DNA of an individual is methylated at one given CpG site, the position is 0% methylated. When all the DNA of the individual is methylated at that given CpG site, the position is 100% methylated. When only of portion, e.g., 50%, 75%, or 80%, of the DNA of the individual is methylated at that CpG site, then the CpG position is said to be 50%, 75%, or 80%, methylated, respectively. The term "methylation status" reflects any relative or absolute amount of methylation of a CpG position. Methylation of CpG positions can be assessed by any method used in the art. The terms "methylation" and "hypermethylation" are used herein interchangeably. When used in reference to a CpG positions, they refer to the methylation status corresponding to an increased presence of 5-methylcytosine at a CpG site within the DNA of a biological sample obtained from a patient, relative to the amount of 5-methylcytosine found at the CpG site within the same genomic position of a biological sample obtained from a healthy individual, or alternatively form an individual suffering from a tumor of a different class or species.

The term "biological sample" is used herein in its broadest sense. In the practice of the present invention, a biological sample is generally obtained from a subject. A sample may be any biological tissue or fluid with which the methylation status of biomarkers of the present invention may be assayed. Frequently, a sample will be a "clinical sample" (i.e., a sample obtained or derived from a patient to be tested). The sample may also be an archival sample with known diagnosis, treatment, and/or outcome history. Examples of biological samples suitable for use in the practice of the present invention include, but are not limited to, bodily fluids, e.g., blood samples (e.g., blood smears), and cerebrospinal fluid, brain tissue samples or bone marrow tissue samples such as tissue or fine needle biopsy samples. Biological samples may also include sections of tissues such as frozen sections taken for histological purposes. The term "biological sample" also encompasses any material derived by processing a biological sample. Derived materials include, but are not limited to, cells (or their progeny) isolated from the sample, as well as nucleic acid molecules (DNA and/or RNA) extracted from the sample. Processing of a biological sample may involve one or more of: filtration, distillation, extraction, concentration, inactivation of interfering components, addition of reagents, and the like.

The method according to the present invention includes a step of "determining the DNA methylation status" of a multitude of independent genomic CpG positions in a biological sample obtained from a patient. Determination of the methylation status may be performed using any method known in the art to be suitable for assessing the methylation of cytosine residues in DNA. Such methods are known in the art and have been described; and one skilled in the art will know how to select the most suitable method depending on the number of samples to be tested, the quantity of sample available, and the like.

Thus, the methylation status of a genomic CpG position or a combination of genomic CpG positions according to the invention can be determined using any of a wide variety of methods that are generally divided into strategies based on methylation-specific PCR (MSP), and strategies employing PCR performed under methylation-independent conditions (MIP). Methylation-independent PCR (MIP) primers are used in most of the available PCR-based methods. They are designed for proportional amplification of methylated and unmethylated DNA. In contrast, methylation-specific PCR (MSP) primers are designed for the amplification of methylated template only.

Examples of methylation-independent PCR based techniques include, but are not limited to, direct bisulfite direct sequencing (Frommer et al., PNAS USA, 1992, 89: 1827-1831), pyrose-quencing (Collela et al., Biotechniques, 2003, 35: 146-150; Uhlmann et al., Electrophoresis, 2002, 23: 4072-4079; Tost et al., Biotechniques, 2003, 35: 152-156), Combined Bisulfite Restriction Analysis or "COBRA" (Xiong et al., Nucleic Acids Res., 1997, 25: 2532-2534), Methylation-Sensitive Single-Nucleotide Primer Extension or "MS-SnuPE" (Gonzalgo et al., Nucleic Acids Res., 1997, 25: 2529-2531), Methylation-Sensitive Melting Curve Analysis or "MS-MSA" (Worm et al., Clin. Chem., 2001, 47: 1183-1189), Methylation-Sensitive High-Resolution Melting or "MS-HRM" (Wojdacz et al., Nucleic Acids Res., 2007, 35:e41), MALDI-TOF mass spectrometry with base-specific cleavage and primer extension (Ehrich et al., PNAS USA, 2005, 102: 15785-15790), and HeavyMethyl (Cottrell et al., Nucleic Acids Res., 2004, 32: e10).

Examples of methylation-specific PCR based techniques include for example methylation specific PCR or "MSP" (Herman et al., PNAS USA, 1996, 93: 9821-9826; Mackay et al., Hum. Genet., 2006, 120: 262-269; Mackay et al., Hum. Genet., 2005, 116: 255-261; Palmisano et al., Cancer Res., 2000, 60: 5954-5958; Voso et al., Blood, 2004, 103: 698-700), MethylLight (Eads et al., Nucleic Acids Res., 2000, 28:e32; Eads et al., Cancer Res., 1999, 59: 2302-2306; Lo et al., Cancer Res., 1999, 59: 3899-3903), Melting curve Methylation Specific PCR or "McMSP" (Akey et al., Genomics, 2002, 80: 376-384), Sensitive Melting Analysis after Real-Time MSP or "SMART-MSP" (Kristensen et al., Nucleic Acids Res., 2008, 36: e42), and Methylation-Specific Fluorescent Amplicon Generation or "MS-FLAG" (Bonanno et al., Clin. Chem., 2007, 53: 2119-2127).

A large number of these methods rely on the prior treatment of DNA with sodium bisulfite. This treatment leads to the conversion of unmethylated cytosine to uracil, while methylated cytosine remains unchanged (Clark et al., Nucleic Acids Res., 1994, 22: 2990-2997). This change in the DNA sequence following bisulfite conversion can be detected using a variety of methods, including PCR amplification followed by DNA sequencing. It is safe to say that the use of bisulfite-converted DNA for DNA methylation analysis has surpassed almost every other methodology for DNA methylation analysis, thereby becoming the gold standard for detecting changes in DNA methylation. The protocol described by Frommer et al. (PNAS USA, 1992, 89: 1827-1831) has been widely used for sodium bisulfite treatment of DNA, and a variety of commercial kits are now available for this purpose.

Thus, in a method according to the invention, the step of determining the methylation status of a gene promoter, or of a combination of gene promoters of the invention, may be performed using any of the techniques described above or any combination of these techniques. One skilled in the art will recognized that when the methylation status of a combination of gene promoters has to be determined, the determinations may be performed using the same DNA methylation analysis technique or different DNA methylation analysis techniques. Other methods include oligonucleotide methylation tiling arrays, BeadChip assays, HPLC/MS methods, methylation-specific multiplex ligation-dependent probe amplification (MS-MPLA), bisulfite sequencing, and assays using antibodies to DNA methylation, i.e., ELISA assays.

The terms "classification rule" or "classifier" refer to a statistical test for classifying biological data of the invention (the methylation data), e.g. in answer to a "classification question." A classification rule can be derived to classify the biological data with respect to various categories. These categories can be associated with different biological activities or different biological states, in the present case the individual tumor species or classes. For example, a set of methylation levels of various CpG positions can be obtained from a biological sample using the afore-mentioned methylation assays, and the set of genomic DNA methylation levels can be classified to one of various types of tumors.

As used herein the term "random forest analysis" refers to a computational method that is based on the idea of using multiple different decision trees to compute the overall most predicted class (the mode). In a specific application, the mode will be either tumor species or class based on how many decision trees predicted the samples to match a specific class. The class predicted by the majority is selected as the predicted class for the sample. The different decision trees used in this algorithm are trained on a randomly generated subset of the training data set and on a randomly selected set of the variables. This is why this algorithm relies on two hyperparameters: the number of random trees to use, and the number of random variables used to train the different trees.

The term "training data set" in context of the invention refers to a set of genomic methylation data of a multitude tumors that were classified by prior art methods, and therefore are of known tumor species.

In some embodiments the method of the invention is performed as an *ex-vivo* or *in-vitro* method.

Most preferably the method according to the invention is used for the classification of brain tumors, therefore, the tumor preferably is a brain tumor, and the tumor species is a brain tumor species. As already noted herein before, brain tumours are characterized by a huge epigenetic variety which has a significant impact on the development of treatment regimes in order to allow for the best treatment of the patient. If the tumor disease is a tumor of the central nervous system (CNS), it is preferred that said tumor species comprises at least 50 different classes of CNS tumors.

The determination of DNA methylation levels in accordance with the method of the invention is performed preferably with a genomic array or chip comprising probes which are specific for the methylation of at least 1000 CpG positions. Preferably is to test as many positions as possible in order to allow for the generation of a highly specific classification. Genome wide DNA methylation assays are therefore preferred, such as the HumanMethylation450k-chip (Illumina^{®}).

The training of the classification-rule according to some embodiments of the invention may comprise a preceding step of selecting CpG position which of all CpG positions used provide the most pure splitting rules, and using said selected CpG positions as a training-data-optimization-set to train the classification-rule.

In other embodiments of the invention the training of the classification-rule may comprise a step of down-sampling for each tumor species the number of boot-strap samples to the minority class, the minority class being the lowest sample size of a tumor species in the training-data-set.

Another embodiment of the invention provides the above method and comprising the further step (d) including the methylation data of the tumor sample as classified in (c) into the training-data-set to obtain an enhanced-training-data-set, and computing an enhanced-classification-rule by random forest analysis based on the enhanced-training-data-set. Optionally the classification of said tumor-sample may be repeated with the enhanced-classification-rule. This embodiment serves the continuous development and improvement of the original training data set. Each further classified tumor species will have a genomic DNA methylation profile that further enhances the classification for that tumor species. Therefore, the invention in one preferred embodiment provides a classification system characterized by a self-learning classification rule.

In order to provide a classification rule with good specificity and sensitivity, the pre-determined methylation data used in context of the present invention includes for each pre-classified tumor species the methylation status/levels at said CpG position of at least one, two, three, four, five, six or more independent samples.

The method of the invention is partly performed digitally and therefore in some embodiments the methylation data is provided in computer-readable form and wherein step (c) is performed *in-silico,* preferably on a digital computer.

Another aspect of the present invention then pertains to a method for stratifying the treatment of a tumor patient, comprising the classification of the tumor species of the tumor of the patient according to a classification method of the invention and stratifying the treatment of the patient in accordance with the diagnosed tumor species.

Yet a further aspect of the invention pertains to a computer-implemented method for generating a classification-rule for aiding the classification of tumor samples in cancer diagnosis, the method comprising providing DNA methylation data of a multitude of independent genomic CpG positions of genomes of a multitude of diverse pre-classified tumor species of the same tumor type (for example brain cancer, lung cancer, leukemia, etc.); computing a random forest of binary decision trees from the DNA methylation data, wherein in each binary decision tree of said random forest each node is a CpG position, and each terminal leave a specific tumor species, and each binary splitting rule is a methylation status at said CpG position.

To learn a classification rule that allows predicting the class assignment of future diagnostic cases the inventor's applied the machine learning algorithm RandomForest (RF; Breiman, 2001). The RF algorithm is a so called ensemble method that combines the predictions of several 'weak' classifiers to achieve improved prediction accuracy. The RF uses binary classification trees (Classification and Regression Trees (CART); Breiman et al., 1983) as 'weak' classifiers. Each of these trees is a sequence of binary splitting rules that are learned by recursive binary splitting. The CART algorithm starts with all samples assigned to a 'root' node and tries to find the variable, e.g., a measured CpG probe, and a corresponding cut-off that results in the purest split into the different classes. To measure this gain in class 'purity' the Gini index, a classical statistical measure for inequality, may be used. To fit a tree the CART algorithm iteratively repeats these step until no further improvements can be made, i.e., only samples of the same class are assigned to the final 'leaf' node, or a pre-specified node size is achieved. To predict the class of a new diagnostic case the binary splitting rules are compared with the new data starting in the root node down to one of the leaf nodes. The tree then predicts or votes for the class dominating that leaf node.

Decision trees have the advantage that they are non-parametric and do not rely on any distributional assumptions. Moreover, trees allow to learn complex non-linear relationships and interactions, they are easy to interpret and can be efficiently fitted in large data sets. The main disadvantages of decision trees is that they often tend overfit the data and that they have a weak prediction performance.

However, to improve the prediction accuracy of a single tree the RF algorithm combines thousands of trees by bootstrap aggregation (bagging). In brief, each tree is fitted using training data sets that are generated by drawing bootstrap samples, i.e., randomly selecting two-third of the data with replacement. In addition, at each node only a random subset of the available variables are used to find an optimal splitting rule. This additional source of randomization allows selecting variables with lower predictive value that would otherwise be ruled out by the most prominent variables. This feature guarantees that the resulting trees are decorrelated, i.e., they use different variables to find an optimal prediction rule. Taking the majority vote over thousands of bootstrap aggregated and decorrelated trees greatly improves the prediction accuracy of the RF. The majority vote, i.e., the proportion of trees voting for a class, can best used as empirical class probabilities or scores that turned out to be are very useful tool for diagnosis.

To validate the resulting RF classifier a repeated five fold cross-validation is applied. In each cross validation the reference set is randomly split into five parts. Then four-fifth of the data is used to train the RF classifier and one-fifth is used for prediction. Currently the estimated test error of the classifier is around 3.1 %.

Some embodiments pertain to the computer implemented method of the invention where the diverse tumor species are selected from metastatic tumors, tumors stemming from specific tissues, tumors in a specific stage, recurrent tumors, tumors having a specific genetic mutation, tumors of patients having different gender, age or genetic background.

The computer-implemented method of the invention may further comprise a preceding step of selecting a set of CpG positions out of all CpG positions in the methylation data, wherein the set of CpG positions comprise CpG positions which provide the most pure splitting rules in the random forest analysis, and using the methylation data corresponding to said set of CpG positions as a training-data-optimization-set to train the classification-rule. This additional step is performed to improve the classifier. In this step the inventors applied a RF using all CpG probes and measured the average gain in the Gini index for each probe. This is a measure for the importance of a probe for the classification. Then the 30,000 probes with highest importance were used to train the final classifier.

Again preferably the methylation data includes for each pre-classified tumor species the methylation status at said CpG position of at least one, two, three, four, five, six or more independent samples.

In addition the method of the invention may comprise a further step of down-sampling for each tumor species the number of bootstrap samples to the minority class, the minority class being the lowest sample size of a tumor species in the methylation data. Down sampling is preferably for a dataset wherein between classes the number of samples varies greatly. The tumors in the example reference set have classes that vary from 6 to almost 200 and this imbalance may cause a bias leading the classifier to preferably vote for large classes. In context of the invention it is preferred to account for this imbalance by down sampling to the minority class, i.e., to fit a tree the number of bootstrap samples drawn from each class is equal to the number of samples in the minority class.

Provided is also a computer-readable storage medium having computer-executable instructions stored, that, when executed, cause a computer to perform a computer implemented method as mentioned herein above. The term "computer-readable storage medium" should be taken to include a single medium or multiple media (e.g., a centralized or distributed database, and/or associated caches and servers) that store the one or more sets of instructions. The term "computer-readable storage medium" shall also be taken to include any medium that is capable of storing or encoding a set of instructions for execution by the machine and that cause the machine to perform any one or more of the methodologies of the present invention. The term "computer-readable storage medium" shall accordingly be taken to include, but not be limited to, solid-state memories, and optical and magnetic media.

The present invention will now be further described in the following examples with reference to the accompanying figures and sequences, nevertheless, without being limited thereto. For the purposes of the present invention, all references as cited herein are incorporated by reference in their entireties. In the Figures:

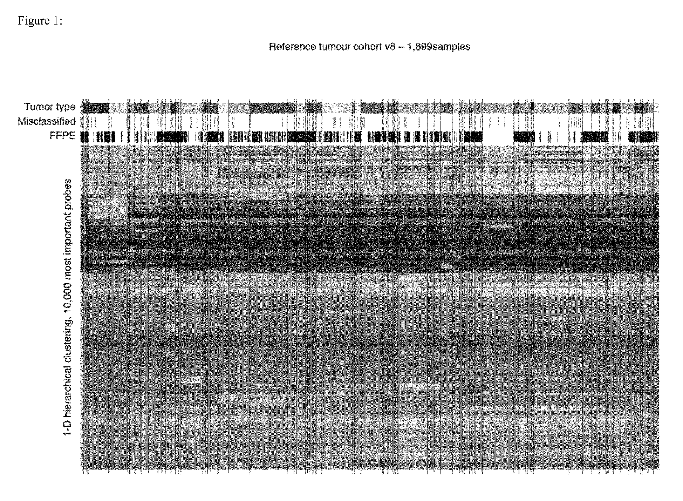

- **Figure 1:**: Heatmap representation of the reference set. The color code indicates the different tumor classes, FFPE and frozen samples as well as samples that are mis-classified in the cross validation. The heatmap shows the methylation profile of 10,000 CpG probes most important for the classification (highest average gain in Gini purity).

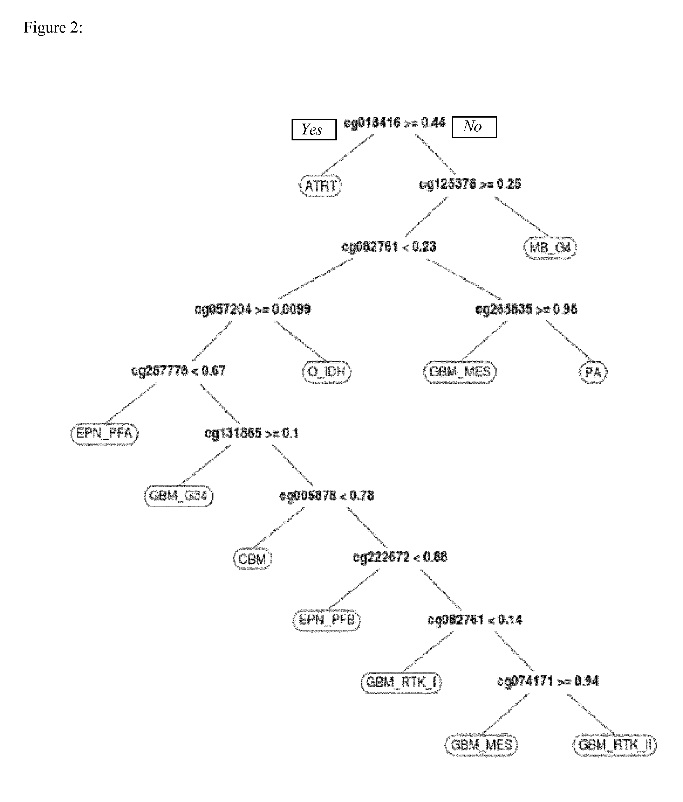

- **Figure 2:**: Example of a binary decision tree. At each node a CpG probe and corresponding cutoff is used to make a binary decision. The final leaf nodes display the abbreviation of the tumor subclass, i.e., EPN_PFA means posterior fossa epen-dymoma subtype A.

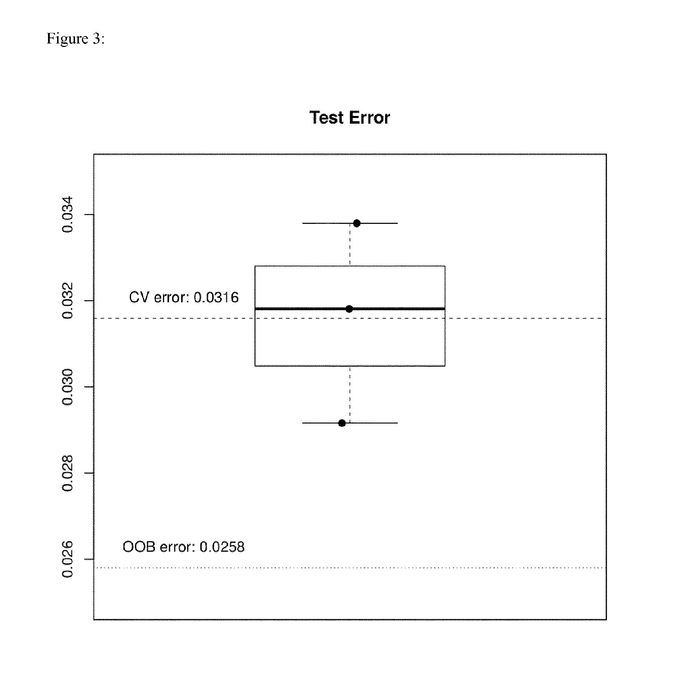

- **Figure 3:**: Median test error estimated by three five-fold cross validation runs.
- **Figure 4:**: The left panel shows a symbolically the histology of a WNT medullablastoma and a Group 3 medullablastoma which are not distinguishable. The right panel shows a multidimensional scaling (MDS) analysis of 107 medulloblastoma samples of all molecular subtypes using the 21,092 most variable CpG probes. WNT medulloblastoma are colored in blue, SHH medulloblastoma in red, Group 3 medulloblastoma in yellow and Group 4 medulloblastoma in green.
- **Figure 5:**: A shows the result of the histology of the patient. B shows the classifier scores. Highlighted is the highest score entry.
- **Figure 6:**: A and B show the result of the histology of the patient. C shows the classifier scores. Highlighted is the highest score entry.

### EXAMPLES

### Materials and Methods

### Infinium Methylation Assay

Genome-wide screening of DNA methylation patterns was performed by using the Infinium HumanMethylation450 BeadChips (Illumina, San Diego, US), allowing the simultaneous quantitative measurement of the methylation status at 485,577 CpG sites. By combining Infinium I and Infinium II assay chemistry technologies, the BeadChip provides coverage of 99% of RefSeq genes and 96 % of CpG islands.

DNA concentrations were determined using PicoGreen (Life Technologies, Darmstadt, Germany). The quality of genomic DNA samples was checked by agarose-gel analysis, and samples with an average fragment size >3kb were selected for methylation analysis. For formalin-fixed paraffin-embedded (FFPE) DNA samples the quality was evaluated by real-time PCR analysis on Light Cycler 480 Real-Time PCR System (Roche, Mannheim, Germany) using the Infinium HD FFPE QC Kit (Illumina). The laboratory work was done in the Genomics and Proteomics Core Facility at the German Cancer Research Center, Heidelberg, Germany (DKFZ).

DNA (500 ng genomic DNA and 250 ng FFPE DNA, respectively) from each sample was bisulfite converted using the EZ-96 DNA Methylation Kit (Zymo Research Corporation, Orange, US) according to the manufacturer recommendations. Bisulfite treatment leads to the deamination of non-methylated cytosines to uracils, while methylated cytosines are refractory to the effects of bisulfite and remain cytosine. After bisulfite conversion, FFPE samples were treated with the Infinium HD DNA Restoration Kit (Illumina) according to the manufacturer recommendations. By using enzymatic reactions, degraded FFPE DNA is restored in preparation for the whole genome amplification.

Each sample was whole genome amplified and enzymatically fragmented following the instructions in the Illumina Infinium HD Assay Methylation Protocol Guide (genomic DNA) or Infinium HD FFPE Methylation Guide (FFPE DNA), respectively. The DNA was applied to Infinium HumanMethylation450 BeadChip and hybridization is performed for 16-24h at 48°C. During hybridization, the DNA molecules anneal to locus-specific DNA oligomers linked to individual bead types. One or two probes are used to interrogate CpG locus, depending on the probe design for a particular CpG site.

Allele-specific primer annealing is followed by single-base extension using DNP- and Biotin-labeled ddNTPs. For Infinium I assay design, both bead types (one each for the methylated and unmethylated states) for the same CpG locus incorporate the same type of labeled nucleotide, determined by the base preceding the interrogated "C" in the CpG locus, and therefore are detected in the same color channel. Infinium II uses only one bead type with a unique type of probe allowing detection of both alleles. The methylated and unmethylated signals are generated respectively in the green and the red channels.

After extension, the array is fluorescently stained, scanned, and the intensities at each CpGs were measured. Microarray scanning was done using an iScan array scanner (Illumina). DNA methylation values, described as beta values, are recorded for each locus in each sample. DNA methylation beta values are continuous variables between 0 and 1, representing the percentage of methylation of a given cytosine corresponding to the ratio of the methylated signal over the sum of the methylated and unmethylated signals.

### Data preprocessing

All data analysis was performed using the open source statistical programming language R (R Core Team, 2014). Raw data files generated by the iScan array scanner were read and preprocessed using the capabilities of the Bioconductor package minfi (Aryee et al, 2014). With the minfi package the same preprocessing steps as recommended in Illumina's BeadStudio software were performed.

In addition, the following filtering criteria were applied: Removal of probes targeting the X and Y chromosomes (n = 11,551), removal of probes containing a single nucleotide polymorphism (dbSNP132 Common) within five base pairs of and including the targeted CpG-site (n = 24,536), and probes not mapping uniquely to the human reference genome (hg19) allowing for one mismatch (n = 9,993). In total, 438,370 probes were kept for analysis.

### Training the classifier

To learn a classification of 1899 samples that were assigned to 72 different brain tumor subtypes the Random Forest (RF) algorithm implemented in the R package randomForest (Liaw and Wiener, 2002) was used. The RF algorithm is a so called ensemble method that combines the predictions of several 'weak' classifiers to achieve improved prediction accuracy. The RF uses binary classification trees (Classification and Regression Trees (CART); Breiman et al., 1983) as 'weak' classifiers. Each of these trees represents a sequence of binary splitting rules that are learned by recursive binary splitting. The CART algorithm starts with all samples assigned to a 'root' node and tries to find the variable, e.g., a measured CpG probe, and a corresponding cutoff that results in the purest split into the different classes. To measure this gain in class 'purity' the Gini index, a classical statistical measure for inequality, is used. To fit a tree the CART algorithm iteratively repeats these step until no further improvements can be made, i.e., only samples of the same class are assigned to the final 'leaf' node, or a pre-specified node size is achieved. To predict the class of a new diagnostic case the binary splitting rules are compared with the new data starting in the root node down to one of the leaf nodes. The tree then predicts or votes for the class dominating that leaf node. However, to improve the prediction accuracy of a single tree the RF algorithm combines thousands of trees by bootstrap aggregation (bagging). In brief, each tree is fitted using training data sets that are generated by drawing bootstrap samples, i.e., randomly selecting two-third of the data with replacement. In addition, at each node only a random subset of the available variables are used to find an optimal splitting rule. To predict the class of a diagnostic samples the RF takes the majority vote of all trees in the forest.

To learn the classification the default parameter settings of the randomForest function were used and 10,000 decision trees were fitted. In addition, to take the highly imbalanced class sizes into account a downsampling strategy was followed, i.e., to fit a decision tree the number of bootstrap samples drawn from each class was equal to the number of samples in the minority class. To further improve prediction performance of the classifier a variable selection was performed, i.e. in a first step the algorithm is used to calculate the variable importance, e.g. the average improvement in Gini purity of a CpG probe when used for a splitting rule. The final classifier was trained using only the 30,000 CpG probes with highest variable importance measure.

### Internal validation

To validate the resulting classifier and estimate its performance in predicting future diagnostic cases a repeated five fold cross-validation was applied. In example, in each cross-validation run the reference set is randomly split into five parts. Then four-fifth of the data is used to train the RF classifier as described above and one-fifth is used for prediction. Currently the estimated test error of the classifier is around 3.1 %.

### Example 1: Distinguishing WNT medullablastoma from Group 3 medullablastoma

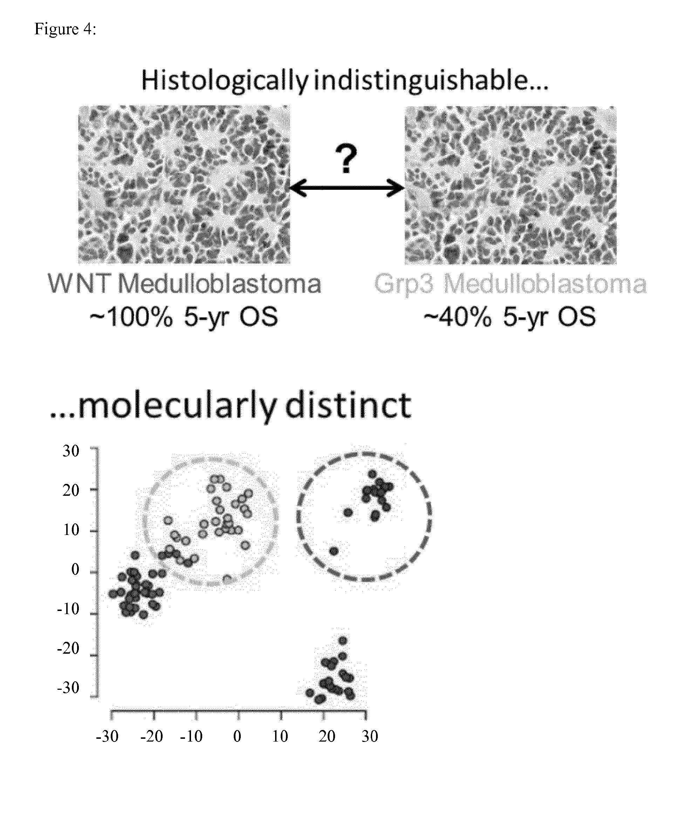

Medulloblastoma is the most common malignant pediatric brain tumor and comprises four distinct molecular variants. These variants are known as WNT, SHH, Group 3, and Group 4. These variants are histologically indistinguishable, but clearly separable by DNA methylation patterns (see figure 4). WNT tumours show activated Wnt signaling pathway and carry a favourable prognosis. SHH medulloblastoma show Hedgehog signaling pathway activation and are known to have an intermediate to good prognosis. While both WNT and SSH variants are molecularly already well characterised, the genetic programs driving the pathogenesis of Group 3 and Group 4 medulloblastoma remain largely unknown.

### Example 2: Change of Diagnosis of a anaplastic astrocytoma WHO III

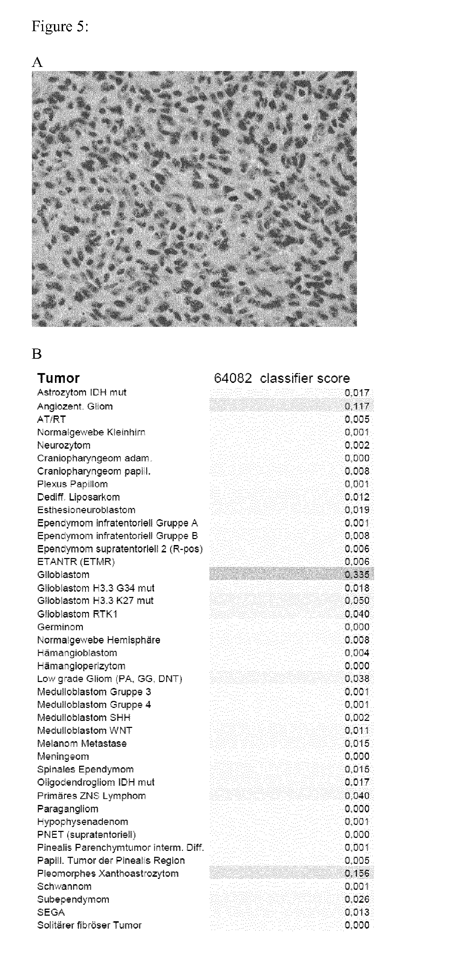

A 1944 born female brain tumor patient was diagnosed based on histology (see figure 5A) to suffer from an anaplastic astrocytoma WHO III. Using the inventive classification procedure, a classifier score of 0.335 changed the diagnosis to Glioblastoma WHO IV (see figure 5B).

### Example 3: Change of Diagnosis of Schwannoma

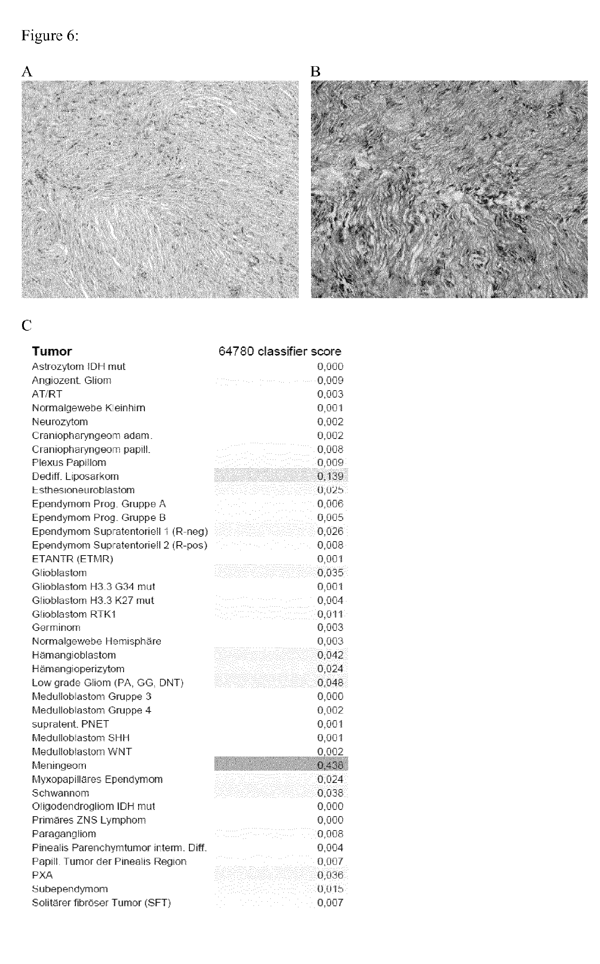

A 1969 born male patient was based on the histology diagnosed with Schwannoma (figure 6A and 6B). The classification procedure of the present invention however was able to diagnose the patient to suffer from Meningeoma WHO I (see figure 6C).

### References:

R Core Team (2014). R: A language and environment for statistical computing. R Foundation for Statistical Computing, Vienna, Austria. URL http://www.R-project.org/.
MJ Aryee, AE Jaffe, H Corrada-Bravo, C Ladd-Acosta, AP Feinberg, KD Hansen, RA Irizarry. Minfi: A flexible and comprehensive Bioconductor package for the analysis of Infinium DNA Methylation microarrays. Bioinformatics 2014, In press. doi: 10.1093/bioinformatics/btu049.
A. Liaw and M. Wiener (2002). Classification and Regression by randomForest. R News 2(3), 18--22.
Bioconductor: Open software development for computational biology and bioinformatics R. Gentleman, V. J. Carey, D. M. Bates, B.Bolstad, M. Dettling, S. Dudoit, B. Ellis, L. Gautier, Y. Ge, and others 2004, Genome Biology, Vol. 5, R80

## Claims

1. An *in-vitro* Method for the classification, stratification and/or diagnosis of a tumor species, the method comprising the steps of
(a) Providing a tumor-sample to be classified obtained from a tumor of a patient and, optionally, isolating genomic DNA therefrom,
(b) Determining DNA methylation status of a multitude of independent genomic CpG positions in the genome of said tumor-sample, and
(c) Classifying the tumor species of the tumor-sample based on the methylation levels as determined in (b) using a classification-rule,
wherein the classification-rule is obtained by random forest analysis of a training-data-set, the training-data-set comprising pre-determined methylation data derived from multitude of pre-classified tumor species, wherein said pre-determined methylation data comprises the methylation status of said CpG positions in the genome of each of said pre-classified tumor species.

2. The method according to claim 1, wherein the tumor is a brain tumor, and wherein the tumor species is a brain tumor species.

3. The method according to claim 1 or 2, wherein determining of DNA methylation levels is performed with a genomic array or chip comprising probes which are specific for the methylation of at least 1000 CpG positions, such as the HumanMethylation450k-chip (Illumina).

4. The method according to any of claims 1 to 3, wherein determining DNA methylation comprises a bisulfite treatment of the DNA.

5. The method according to any of claims 1 to 4, wherein training of the classification-rule comprises a preceding step of selecting CpG position which of all CpG positions used provide the most pure splitting rules, and using said selected CpG positions as a training-data-optimization-set to train the classification-rule.

6. The method according to any of claims 1 to 5, wherein training of the classification-rule comprises a step of down-sampling for each tumor species the number of boot-strap samples to the minority class, the minority class being the lowest sample size of a tumor species in the training-data-set.

7. The method according to any of claims 1 to 6, comprising the further step (d) including the methylation data of the tumor sample as classified in (c) into the training-data-set to obtain an enhanced-training-data-set, and computing an enhanced-classification-rule by random forest analysis based on the enhanced-training-data-set.

8. The method according to any one of claims 1 to 7, wherein the pre-determined methylation data includes for each pre-classified tumor species the methylation status at said CpG position of at least two or more independent samples.

9. An *in-vitro* method for stratifying the treatment of a tumor patient, comprising the classification of the tumor species of the tumor of the patient according to a method of any of claims 1 to 8, and stratifying the treatment of the patient in accordance with the diagnosed tumor species.

10. A computer-implemented method for generating a classification-rule for aiding the classification of tumor samples in cancer diagnosis, the method comprising providing DNA methylation data of a multitude of independent genomic CpG positions of genomes of a multitude of diverse pre-classified tumor species of the same tumor type; computing a random forest of binary decision trees from the DNA methylation data, wherein in each binary decision tree of said random forest each node is a CpG position, and each terminal leave a specific tumor species, and each binary splitting rule is a methylation status at said CpG position.

11. The computer-implemented method according to claim 10, wherein the diverse tumor species are selected from metastatic tumors, tumors stemming from specific tissues, tumors in a specific stage, recurrent tumors, tumors having a specific genetic mutation, tumors of patients having different gender, age or genetic background.

12. The computer-implemented method according to claim 10 or 11, comprising a further preceding step of selecting a set of CpG positions out of all CpG positions in the methylation data, wherein the set of CpG positions comprise CpG positions which provide the most pure splitting rules in the random forest analysis, and using the methylation data corresponding to said set of CpG positions as a training-data-optimization-set to train the classification-rule.

13. The computer-implemented method according to any of claim 10 to 12, wherein the methylation data includes for each pre-classified tumor species the methylation status at said CpG position of at least one, two, three, four, five, six or more independent samples.

14. The computer-implemented method according to any of claim 10 to 13, comprising a step of down-sampling for each tumor species the number of bootstrap samples to the minority class, the minority class being the lowest sample size of a tumor species in the methylation data.

15. A Computer-readable storage medium having computer-executable instructions stored, that, when executed, cause a computer to perform a method according to any of claims 10 to 14.
